Europäisches Patentamt

European Patent Office

Office européen des brevets

(19)

(11) Veröffentlichungsnummer: **0 374 086**

**A1**

## (12) EUROPÄISCHE PATENTANMELDUNG

(21) Anmeldenummer: **89730218.8**

(22) Anmeldetag: **07.12.89**

(51) Int. Cl.5: **A61B 17/14**

(30) Priorität: **14.12.88 DE 3842645**

(43) Veröffentlichungstag der Anmeldung:
**20.06.90 Patentblatt 90/25**

(84) Benannte Vertragsstaaten:
**AT BE CH DE ES FR GB GR IT LI NL SE**

(71) Anmelder: **MECRON MEDIZINISCHE
PRODUKTE GMBH
Nunsdorfer Ring 23-29
D-1000 Berlin 48(DE)**

(72) Erfinder: **Schmidt, Joachim, Dr. med.
Mohnweg 18
D-5000 Köln 41(DE)**

(74) Vertreter: **Christiansen, Henning, Dipl.-Ing.
Patentanwalt CHRISTIANSEN Paceliiallee
43/45
D-1000 Berlin 33(DE)**

(54) **Sägelehrensystem.**

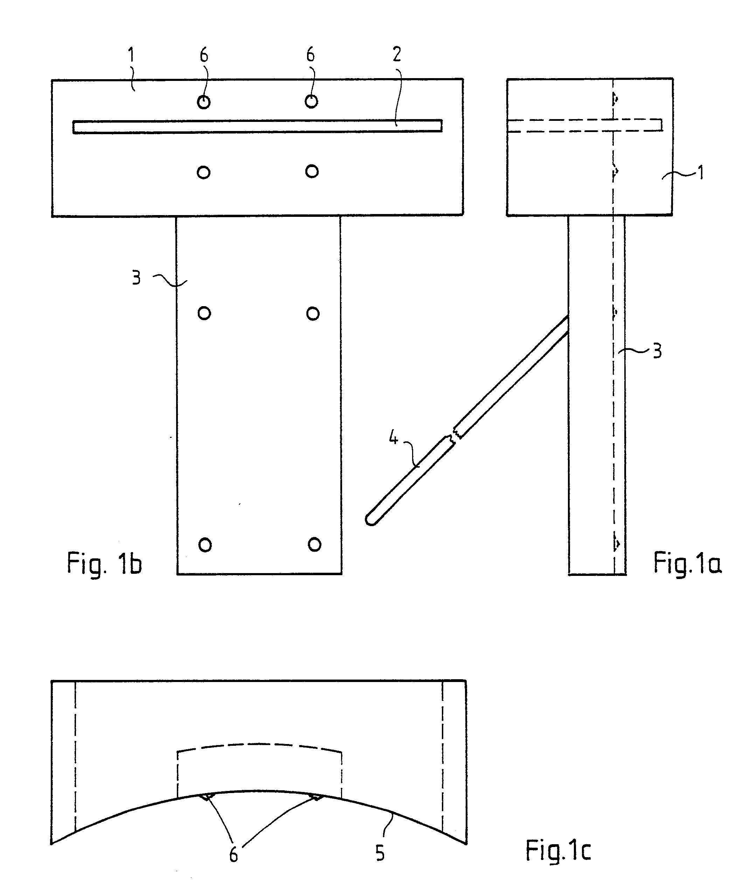

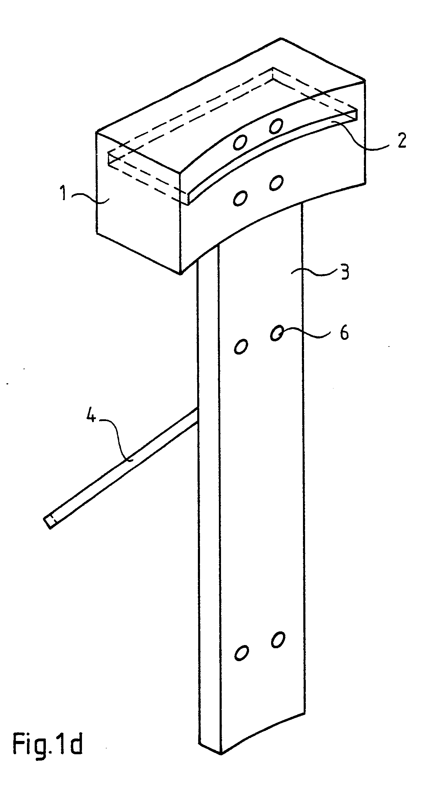

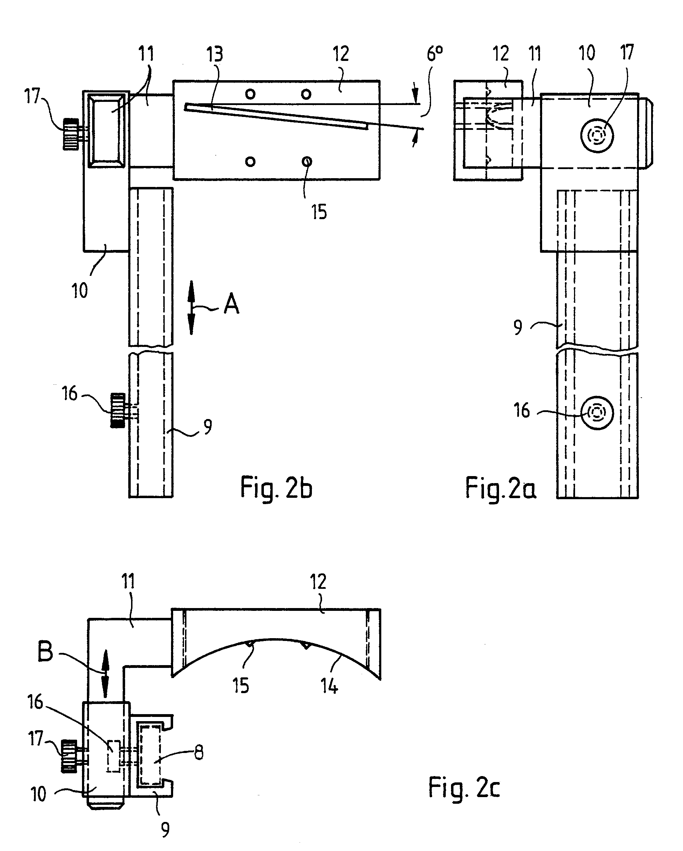

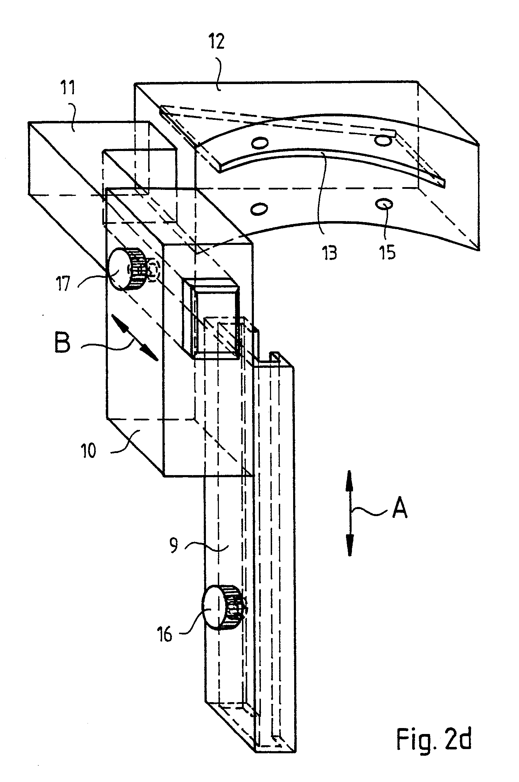

(57) Sägelehrensystem zur Verwendung bei intertrochanteren Umstellungsosteotomien, bei dem eine erste an ihrer Auflageseite (5) hohlzylindrisch gewölbte, auf den Femurschaft flächig auflegbare Sägelehre (Fig. 1) mit einem senkrecht zur Femurschaftachse ausgerichteten ersten Sägespalt (2) versehen ist, und eine zweite an ihrer Auflageseite (14) hohlzylindrisch gewölbte, auf den Femurschaft flächig auflegbare Sägelehre (Fig. 2) mit einem bei zusammenfallenden Achsen der Hohlzylinder um 6° zum ersten Sägespalt (2) geneigten zweiten Sägespalt (13) versehen ist.

Fig. 2d

EP 0 374 086 A1

Xerox Copy Centre

## Sägelehrensystem

Die Erfindung betrifft ein Sägelehrensystem der im Oberbegriff des Anspruchs 1 angegebenen Art.

Bei intertrochanteren Umstellungsosteotomien versprechen große spongiöse Knochenflächen eine gute knöcherne Durchbauung. Trotzdem kommt es gelegentlich zu einer verzöger ten Knochenheilung, die durch Knochenresorption im Osteotomiebereich verursacht wird. Die Resorption ist die Folge von Mikrobewegungen an der Kontaktfläche als Ausdruck einer unzureichenden interfragmenteren Kompression.

Bei der heute üblichen Komprimierung der Fragmente durch die Winkelplatte mit Hilfe des Plattenspanners oder nach dem Gleitlochplattenprinzip muß es durch die exzentrische Lage der Platte überwiegend zu einer Kompression der lateralen Kortikalis kommen. Die mediale Kortikalis gerät hingegen unter Zugbelastung. Das Prinzip der Plattenüberbiegung wird in der Regel nicht berücksichtigt. Es handelt sich um eine Zuggurtungsosteosynthese.

In der Literatur sind verschiedene Angaben darüber zu finden, wie eine interfragmentere Kompression bei intertrochanteren Osteotomien zu erreichen ist. Bei dem Vorgehen nach Buchner (Russ, G.M.: Z. "Orthopädie", 112, 1974, S. 348-350) wird die Kompression beim Einschlagen der Platte durch einen nach medial zunehmenden Abstand zwischen Klingenlager und Osteotomiefläche erreicht. Das Nachschlagen der bereits distal fixierten Platte setzt die Osteotomiefläche unter Druck. R. Schneider ("Die intertrochantere Osteotomie bei Coxarthrose", Springer-Verlag, Berlin, 1979) empfiehlt schräg stehende Osteotomieflächen, die nach dem Anziehen der proximalen Schraube unter Druck geraten. Heisel (Z. "Orthpädie", 122, 1984, S. 705-715) schlägt vor, daß Prinzip der Plattenüberbiegung nach Bagby zu berücksichtigen, um ein mediales Aufklaffen der Osteotomie zu vermeiden. Bagby (J. "Surgery", 95, 1958, S. 761-771) beschrieb die Plattenüberbiegung bei Schaftfrakturen. Allen diesen Methoden haftet jedoch der Nachteil an, daß ihre Wirkung nicht exakt beurteilbar ist, und daß sie darüber hinaus teilweise starke Scherbeanspruchungen bewirken.

Der Erfindung liegt die Aufgabe zugrunde, eine Vorrichtung zur intraoperativen Entnahme eines Knochenkeiles mit einem vorgebenen Keilwinkel zu schaffen, so daß eine optimale Vorspannkraft F einstellbar ist.

Diese Aufgabe wird mit den kennzeichnenden Merkmalen des Anspruchs 1 gelöst.

Das erfindungsgemäße aus zwei einzelnen Sägelehren bestehende Sägelehrensystem gestattet eine schnelle und exakte Ausführung von zwei Schnitten, mit denen der proximale Femur zunächst senkrecht zur Schaftachse und danach mit einem Winkel von insbesondere 6° zum ersten Schnitt durchtrennt wird. Die zweite Sägelehre ist dabei relativ zur Lage des ersten Schnittes genau positionierbar.

Die optimale Durchbauung der Osteotomie erfordert eine optimale interfragmentere Druckverteilung. Dieses Ziel läßt sich über ein exakt in Grad bestimmtes, damit quantifizierbares, Auseinanderklaffen der Osteotomieflächen mit einem nach lateral offenen Winkel a erreichen. Der für die geplante Umstellung notwendige Knochenkeil wird so dimensioniert, daß dieses laterale Auseinanderklaffen nach Abschluß der Umstellung vor dem Spannvorgang verbleibt, d.h. der entnommene Varisationskeil ist um $\alpha$ kleiner, der Valgisationskeil um a größer. Bei Beginn des Spannvorganges mit der Spannkraft F gerät auf diese Weise zunächst die mediale Kortikalis unter Druck. Unter Ausnutzung der elastischen und biomechanischen Eigenschaften einer Winkelplatte wird es bei zunehmendem Spannvorgang zu einer Kippbewegung des proximalen Fragmentes um einen Drehpunkt in der medialen Kortikalis kommen, bis der Osteotomiespalt geschlossen ist und eine gleichmäßige Kompression medial und lateral erreicht wird. Durch diese Bewegung wird eine Scherbelastung vermieden.

Als Ergebnis experimenteller Untersuchungen ergab sich, daß sowohl in Ruhe als auch unter Simulation einer statischen Belastung eine gleichmäßigere interfragmentere Kompressionsverteilung bei intertrochanteren Umstellungsosteotomien erreicht wird, wenn die Osteotomieflächen vor Beginn des Spannvorganges mit F = 1000 N mit einem nach lateral offenen Winkel von $\alpha = 6°$ auseinanderklaffen. Das Prinzip der Zuggurtungsosteosynthese wird dabei durch das Prinzip einer Kompressionsosteosynthese optimiert. Zugbelastung und Spannungsspitzen mit pathologischem Knochenabbau werden reduziert. Relativbewegungen lassen sich vermeiden.

Der Winkel von $\alpha = 6°$ läßt sich mit zwei Sägelehren problemlos intraoperativ realisieren. Der Knochen wird mittels einer ersten Sägelehre exakt senkrecht zum distalen Fragment durchtrennt. Anschließend wird das Plattensitzinstrument mit einem um 6° kleineren Winkel gegenüber dem Schenkelschaft eingeschlagen als für die geplante Umstellung notwendig, weil sich die Platte bei dem Spannvorgang um 6° aufbiegt, d.h. valgisiert. Nach Positionierung der Winkelplatte wird eine zweite Sägelehre auf den Plattenschaft aufgesteckt. Diese ermöglicht die Entnahme eines Korrekturkeiles am proximalen Fragment unter Berücksichtigung des Winkels $\alpha = 6°$. Die Sägerichtung verläuft senkrecht zum Plattenschaft minus 6°. Dadurch verbleibt ein laterales Klaffen der Osteotomieflächen von 6°. Anschließend wird die Platte mit einer Schraube durch das oberste Langloch am Oberschenkelschaft fixiert und dann mit dem Plattenspanner gespannt. Der Abschluß der Montage erfolgt in herkömmlicher Weise.

Die Sägelehren sind mit konkav gewölbten Unterseiten versehen, die eine exakte Auflage auf dem Femurschaft garantieren. Zur Verschiebesicherung dienen Metallnoppen, die im Periost haften und/oder als Klemmschrauben ausgebildete Rändelschrauben.

Die Länge der Auflagefläche insbesondere der ersten Sägelehre ist durch die Größe der Operationswunde limitiert, jedoch für die parallele Einstellung ausreichend bemessen, wenn die Länge der Auflagefläche größer ist als die Länge des Sägespaltes. Die erste Sägelehre wird mit einem Griff manuell gesichert.

Die zweite Sägelehre wird nach dem Anbringen einer Winkelplatte mit einem Gleitstück auf den Winkelplattenschaft aufgeschoben und vorzugsweise mittels einer Rändelschraube arretiert. An dem Gleitstück ist ein Sägeblock mit Sägespalt über ein längenverstellbares, ebenfalls vorzugsweise arretierbares Bauteil und ein Kniestück befestigt. Die Bewegungsrichtungen des Gleitstückes und des längenverstell baren Bauteils stehen senkrecht aufeinander. Dadurch wird eine genaue Positionierung der zweiten Sägelehre gegenüber dem mit der ersten Sägelehre durchgeführten ersten Osteotomieschnitt möglich. Das Kniestück zwischen dem höhenverstellbaren Bauteil und dem Sägeblock sorgt dafür, daß der Anlagebereich der zweiten Sägelehre an den Femurschaft um genau 90° versetzt gegenüber dem Anlagebereich des Winkelplattenschaftes liegt. Die Sägerichtung entspricht, dem in den Sägeblock eingefrästen Sägespalt gemäß, $90° - 6° = 84°$ zum Femurschaft, so daß die Entnahme eines Korrekturkeiles am proximalen Fragment erfolgen kann und ein laterales Auseinanderklaffen der Osteotomieflächen von 6° verbleibt.

Die Anwendung des Sägelehrensystems zur Behandlung intertrochanterer Osteotomien bewirkt eine Verminderung der Pseudarthrosebildung und der verzögerten Knochenheilungen sowie eine größere Sicherheit für diese übungsstabile Osteosynthese bei der postoperativen Krankengymnastik und Mobilisation.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1a eine schematisierte Seitenansicht eines vorteilhaften Ausführungsbeispiels der ersten Sägelehre,

Figur 1b eine entsprechende Ansicht der auf den Femurschaft aufzulegenden Unterseite der ersten Sägelehre gemäß Figur 1a,

Figur 1c eine Stirnansicht der ersten Sägelehre gemäß Figur 1a,

Figur 1d eine perspektivische Ansicht der ersten Sägelehre gemäß Figuren 1a bis 1c,

Figur 2a eine schematisierte Seitenansicht eines Ausführungsbeispiels der zweiten Sägelehre,

Figur 2b eine entsprechende Draufsicht der zweiten Sägelehre,

Figur 2c eine entsprechende Stirnansicht der zweiten Sägelehre,

Figur 2d eine perspektivische Darstellung der zweiten Knochenplatte,

Figur 3a die Lage des Osteotomiewinkels vor der Kompression und

Figur 3b die interfragmentare Kompressionsverteilung bei einem Osteotomiewinkel von 6°.

Der experimentell als optimal ermittelte Keilwinkel von $\alpha = 6°$ läßt sich mit zwei nacheinander anzusetzenden Sägelehren problemlos intraoperativ einstellen. Der Femur wird mit Hilfe einer ersten Sägelehre exakt senkrecht zu seiner Schaftachse durchtrennt. Die in den Figuren 1a bis 1d in verschiedenen Ansichten dargestellte erste Sägelehre besteht im wesentlichen aus einem Sägeblock 1 mit einem Sä gespalt 2, einer Auflage 3 und einem Haltegriff 4, wobei der Sägespalt 2 exakt senkrecht zur Längsrichtung der Auflage 3 und folglich senkrecht zur Schaftachse in den Sägeblock 1 eingefräst ist. Die Auflage 3 ist an ihrer Unterseite 5 hohlzylindrisch gewölbt und damit der Knochenform angepaßt. Um eine sichere Anlage an den Femurschaft zu garantieren, ist die Längsausdehnung der Auflage 3 maximiert. Eine Limitierung ergibt sich aus der Größe des Operationsgebietes. Zur Sicherung der Auflage 3 gegen unbeabsichtigte Verschiebungen entlang der Knochenoberfläche ist die Unterseite 5 der Auflage 3 mit mehreren, gleichmäßig verteilten, im Periost haftenden Metallnoppen 6 versehen. Die Auflage 3 trägt den Sägeblock 1 mit dem Sägespalt 2. Ein Haltegriff 4, der an der Oberseite der Auflage 3 montiert ist, erlaubt eine zusätzliche manuelle Sicherung des ersten Sägeblockes gegen unbeabsichtigtes Verrutschen insbesondere während des Sägevorganges.

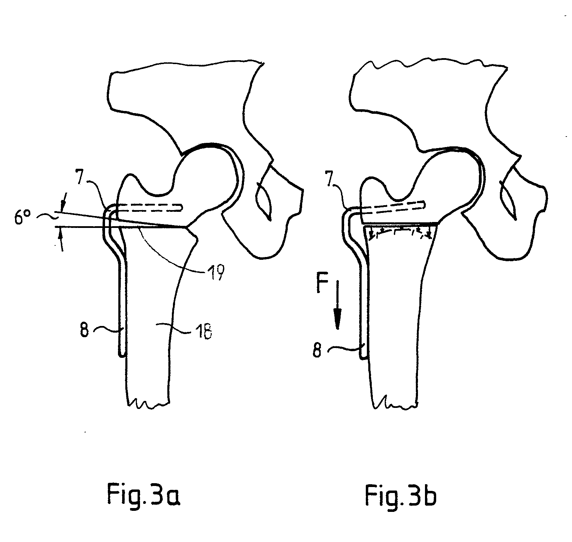

Nach dem Durchtrennen des Knochens, wobei der Sägespalt 2 als Schablone dient, wird die erste Sägelehre entfernt und ein Plattensitzinstrument wird mit einem um 6° kleineren Winkel gegenüber dem Schenkelschaft eingeschlagen als für die geplante Umstellung notwendig, weil sich - wie aus den Figuren 3a und 3b ersichtlich - eine einzusetzende Winkelplatte 7 während des Spannens um 6° aufbiegt.

Nach dem Positionieren der Winkelplatte 7 wird eine zweite Sägelehre auf den Plattenschaft 8 der Winkelplatte 7 aufgeschoben.

Die in den Figuren 2a bis 2d in verschiedenen Ansichten wiedergegebene zweite Sägelehre dient der Entfernung des für die Umstellung notwendigen Keiles aus dem Knochen unter Berücksichtigung eines Keilwinkels von 6°. Sie unterscheidet sich von der ersten Sägelehre vor allem durch die Art ihrer Positionierung und durch die Lage ihres Sägespaltes. Die zweite Sägelehre besteht im wesentlichen aus einem Gleitstück 9, das auf den Plattenschaft 8 (in Figur 2c gestrichelt dargestellt) der Winkelplatte 7 entsprechend dem Pfeil A aufgeschoben wird, einem entsprechend Pfeil B längenverstellbaren Bauteil 10, einem Kniestück 11 und einem Sägeblock 12 mit schräg eingefrästem Sägespalt 13. Die auf der Knochenoberfläche aufzulegende Unterseite 14 des Sägeblocks 12 ist, der Knochenform entsprechend, hohlzylindrisch ausgeformt und mit mehreren Metallnoppen 15 zur Verbesserung der Haftung versehen. Das Gleitstück 9 ist mit dem längenverstellbaren Bauteil 10 auf eine Weise verbunden, daß zueinander und zu dem mittels der ersten Sägelehre geschaffenen Osteotomiespalt senkrechte Bewegungen möglich sind. Über ein rechtwinkliges Kniestück 11, das einerseits mit dem längenverstellbaren Bauteil 10 und andererseits mit dem Sägeblock 12 verbunden ist, läßt sich der Sägeblock 12 durch Betätigung des längenverstellbaren Bauteiles 10 auf den Knochen absenken und durch Verschiebung des Gleitstükkes 9 entlang des Plattenschaftes 8 der Winkelplatte 7 soweit längsverschieben, daß der Sägespalt 13 des Sägeblokkes 12 exakt zu dem bereits bestehenden Osteotomiespalt positioniert ist. Die eingestellte Relativlage zwischen dem Gleitstück 9 und dem Plattenschaft 8 wird mittels einer gerändelten Klemmschraube 16 fixiert. Eine weitere gerändelte Klemmschraube 17 ist zur Fixation des längenverstellbaren Bauteiles 10 auf dem Kniestück 11 vorgesehen.

Die Entnahme des Korrekturkeiles erfolgt am proximalen Fragment. Die Sägerichtung entspricht einem Winkel von 90° - 6° = 84° zum Winkelplattenschaft 8. Dadurch verbleibt ein laterales Klaffen der Osteotomieflächen von 6°.

Die Figuren 3a und 3b veranschaulichen die Wirkung des nachfolgenden Spannvorganges unter Ausnutzung des mit den erfindungsgemäßen Sägelehren im Femur 18 erzeugten Spaltes 19. Die Winkelplatte 7 mit dem Plattenschaft 8 wird dazu mit einer in ein oberstes (nicht dargestelltes) Langloch eingesetzten Schraube fixiert. Mittels eines üblichen (ebenfalls nicht dargestellten) Plattenspanners wird dann werden dann die Knochenteile von der in Figur 3a dargestellten Position mit klaffendem Spalt in die in Figur 3b dargestellte Position mit geschlossenem Spalt überführt. Die Vorspannungskraft beträgt ca. 1000 N. Der abschließende Fixierung erfolgt in herkömmlicher Weise. Die Spannvorrichtung ist nicht Teil der Erfindung. Die Darstellung gemäß Figuren 3a und 3b dient lediglich zur Verdeutlichung des chirurgischen Verfahrens bei dem die erfindungsgemäßen Sägelehren eingesetzt werden, um das Verständnis ihrer Funktion zu erleichtern.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Ansprüche

1. Sägelehrensystem zur Verwendung bei intertrochanteren Umstellungsosteotomien, **dadurch gekennzeichnet,** daß eine erste an ihrer Auflageseite (5) hohlzylindrisch gewölbte, auf den Femurschaft flächig auflegbare Sägelehre (Fig. 1) mit einem senkrecht zur Femurschaftachse ausgerichteten ersten Sägespalt (2) versehen ist, daß eine zweite an ihrer Auflageseite (14) hohlzylindrisch gewölbte, auf den Femurschaft flächig auflegbare Sägelehre (Fig. 2) mit einem bei zusammenfallenden Achsen der Hohlzylinder um 6° zum ersten Sägespalt (2) geneigten zweiten Sägespalt (13) versehen ist.

2. Sägelehrensystem nach Anspruch 1 , **dadurch gekennzeichnet,** daß die Auflageflächen (5 und 14) der Sägelehren auf den Femurschaft mit erhabenen Metallnoppen (6 und 15) versehen sind.

3. Sägelehrensystem nach einem der vorangehenden Ansprüche, **dadurch gekennzeichnet,** daß die Länge der Auflagefläche (5) der ersten Sägelehre größer als die Länge des Sägespaltes (2) ist.

4. Sägelehrensystem nach einem der vorangehenden Ansprüche, **dadurch gekennzeichnet,** daß die erste Sägelehre einen Haltegriff (4) aufweist.

5. Sägelehrensystem nach einem der vorangehenden Ansprüche, **dadurch gekennzeichnet,** daß die zweite Sägelehre (Fig. 2) ein Gleitstück (9) aufweist, das auf den Schaft (8) einer Winkelplatte (7) aufschiebbar ist, wobei die Ebene der Auflagefläche (14) der zweiten Sägelehre und die Ebene der Erstreckung der Winkelplatte (7) senkrecht zueinander stehen und die Richtung der Achse der hohlzylindrischen Ausnehmung der Auflagefläche (14) und die Richtung der Winkelplatte (7) im wesentlichen parallel verlaufen.

6. Sägelehrensystem nach Anspruch 5, **dadurch gekennzeichnet,** daß der räumliche Abstand der Winkelplatte (7) und der Achse der hohlzylindrischen Ausnehmung mittels eines teleskopartig oder entsprechend gegeneinander verschiebbare Elemente aufweisenden in der Länge veränderbaren Bauteiles (10) positionierbar ist.

7. Sägelehrensystem nach Anspruch 5, dadurch gekennzeichnet , daß Fixationsmittel zur Arretierung des Gleitstücks (9) an der Winkelplatte (7) oder/und des längenveränderbaren Bauteiles (10) vorgesehen sind.

8. Sägelehrensystem nach Anspruch 7 , **dadurch gekennzeichnet,** daß zur Arretierung mindestens eine Rändelschraube (16, 17) vorgesehen ist.

ME 38.17-EU

1    6    6    2

1

3

Fig. 1b

4

Fig.1a

3

Fig.1c

6    5

ME 38.17-EU

Fig.1d

ME 38.17-EU

Fig. 2b

Fig. 2a

Fig. 2c

ME 38.17- EU

Fig. 2d

ME 38.17 – EU

Fig.3a          Fig.3b

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 211 153  (P. HOLZHAUSER) <br> * Ganzes Dokument * <br> --- | 1-8 | A 61 B  17/14 |
| A | WO-A-8 701 579  (J.M. AUBANIAC) <br> * Seite 11, Zeile 12 - Seite 12, Zeile 25; Figuren 12-13 * <br> --- | 1-2 | |
| A | CH-A- 511 016  (OSTEO) <br> * Spalte 3, Zeilen 11-36; Figuren 2-3 * <br> --- | 1,3 | |
| A | US-A-4 718 413  (W. JOHNSON) <br> * Spalte 3, Zeilen 12-35; Anspruch 10; Figur 1 * <br> ----- | 1,4 | |

**RECHERCHIERTE SACHGEBIETE (Int. Cl.5)**

A 61 B
B 23 D

Der vorliegende Recherchenbericht wurde für alle Patentansprüche erstellt

| Recherchenort | Abschlußdatum der Recherche | Prüfer |
|---|---|---|
| DEN HAAG | 28-02-1990 | NICE P.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)